# EUROPEAN PATENT APPLICATION

(11) **EP 4 260 880 A1**
(43) Date of publication of application: **18.10.2023**
(21) Application number: 22167915.2
(22) Date of filing: 12.04.2022
(51) Int. Cl.: A61L 9/20, F21S 8/04

(54) **BEAM SHAPING OF 214 NM ZINC DISCHARGE LAMPS**

(71) Applicant: Signify Holding B.V., 5656 AE Eindhoven (NL)
(72) Inventor: The designation of the inventor has not yet been filed
(74) Representative: Verweij, Petronella Daniëlle

(57) **Abstract**

The invention provides a radiation generating system (1000) comprising (i) an optical element assembly (400), and (ii) a light generating device (100), wherein:
- the light generating device (100) is configured to generate device radiation (101) having one or more wavelengths selected from the wavelength range of 100-380 nm; the light generating device (100) comprises an elongated element (150) having an element length (L1), wherein the elongated element (150) comprises one or more radiation emitting faces (151), over at least part of the element length (L1), from which during operation of the light generating device (100) the device radiation (101) emanates;
- the optical element assembly (400) comprises a first hollow reflective element (410) and a second hollow reflective element (420), wherein for the hollow reflective elements (410,420) applies: (a) the hollow reflective element (410,420) has a first end (411,421) and a second end (412,422); (b) the hollow reflective element (410,420) tapers from the second end (412,422) to the first end (411,421); (c) the hollow reflective element (410,420) has an optical axis (O₁,O₂); and (d) the hollow reflective element (410,420) comprises one or more focal points (F₁,F₂), configured in a plane (P₁,P₂) perpendicular to the optical axis (O₁,O₂);
- the first ends (411,421) of the hollow reflective elements (410,420) are directed to each other or coincide, and the planes (P₁,P₂) do not coincide;
- the elongated element (150) is (i) configured at least partly coinciding with the optical axes (O₁,O₂) or (ii) configured between the first ends (411,421) and perpendicular to the optical axes (O₁,O₂);
- the radiation generating system (1000) is configured to generate system radiation (1001) comprising at least part of the device light (101).

## Description

### FIELD OF THE INVENTION

The invention relates to a radiation generating system to provide ultraviolet radiation. The invention further relates to a method for treating a gas or surface (external to such radiation generating system) with the ultraviolet radiation.

### BACKGROUND OF THE INVENTION

Radiation generating systems providing ultraviolet radiation are known in the art. For instance, US20210386901A1 describes a lighting system for disinfection and decontamination of an air stream that is moved by an air circulation means into the interior of the lighting system through an inlet, filtered by an air filtration means, and irradiated by ultraviolet light emitted by ultraviolet radiation sources, and lastly exhausted via an outlet, and thereby the lighting system disinfects and decontaminates the air and adjacent surfaces. The ultraviolet light is contained within the system, so that a person adjacent to or present within a predetermined distance is not exposed to unsafe ultraviolet radiation. The radiation sources can be UV, UVC, Near UVA and Visible Light LEDs with a long-lasting rated life. The lighting system may be operated by a smart light switch, wireless communication of a mobile device, a smart home control appliance, and/or built-in motion sensors and software.

### SUMMARY OF THE INVENTION

UV light has been used for disinfection for over 100 years. Wavelengths between about 190 nm and 300 nm may be strongly absorbed by nucleic acids, which may result in defects in an organism's genome. This may be desired for inactivating (killing), bacteria and viruses, but may also have undesired side effects for humans. Therefore, the selection of wavelength of radiation, intensity of radiation and duration of irradiation may be limited in environments where people may reside such as offices, public transport, cinema's, restaurants, shops, etc., thus limiting the disinfection capacity. Especially in such environments, additional measures of disinfection may be advantageous to prevent the spread of bacteria and viruses such as influenza or novel (corona) viruses like COVID-19, SARS, and MERS.

It appears desirable to produce systems, that provide alternative ways for air treatment, such as disinfection. Further, existing systems for disinfection may not easily be implemented in existing infrastructure, such as in existing buildings like offices, hospitality areas, etc. and/or may not easily be able to serve larger spaces. This may again increase the risk of contamination. Further, incorporation in HVAC systems may not lead to desirable effects and appears to be relatively complex. Further, existing systems may not be efficient, or may be relatively bulky, and may also not easily be incorporated in functional devices, such as e.g. luminaires.

Other disinfection systems may use one or more anti-microbial and/or antiviral means to disinfect a space or an object. Examples of such means may be chemical agents which may raise concerns. For instance, the chemical agents may also be harmful for people and pets.

In embodiments, the disinfecting light, may especially comprise ultraviolet (UV) radiation (and/or optionally violet radiation), i.e., the light may comprise a wavelength selected from the ultraviolet wavelength range (and/or optionally the violet wavelength range). However, other wavelengths are herein not excluded. The ultraviolet wavelength range is defined as light in a wavelength range from 100 to 380 nm and can be divided into different types of UV light / UV wavelength ranges (Table 1). Different UV wavelengths of radiation may have different properties and thus may have different compatibility with human presence and may have different effects when used for disinfection (Table 1).

**Table 1: Properties of different types of UV, violet, and NIR wavelength light**

| Name | Short name | Wavelength (nm) | (Relative) sterilization effectiveness | | Safe Radiation | Vitamin D generation | Ozone generation |
|---|---|---|---|---|---|---|---|
| | | | Bacteria | Viruses | | | |
| Near Infrared (deep red) | NIR | 780-950 | + | +/- | +++ | | |
| Violet | V | 380-420 | +/- | - | + | | |
| Ultra-violet A | UV-A | 320-380 | + | - | + | | |
| Ultra-violet B | UV-B | 280-320 | + | +/- | +/- | + | |
| Near ultraviolet C | Near UV-C | 230-280 | ++ | ++ | - | | |
| Far ultra-violet C | Far UV-C | 190-230 | +++ | +++ | + | | +/- |
| Extreme ultraviolet C | Extreme UV-C | 100-190 | +++ | +++ | - | | + |

Each UV type / wavelength range may have different benefits and/or drawbacks. Relevant aspects may be (relative) sterilization effectiveness, safety (regarding radiation), and ozone production (as result of its radiation). Depending on an application a specific type of UV light or a specific combination of UV light types may be selected and provides superior performance over other types of UV light. UV-A may be (relatively) safe and may inactivate (kill) bacteria, but may be less effective in inactivating (killing) viruses. UV-B may be (relatively) safe when a low dose (i.e. low exposure time and/or low intensity) is used, may inactivate (kill) bacteria, and may be moderately effective in inactivating (killing) viruses. UV-B may also have the additional benefit that it can be used effectively in the production of vitamin D in a skin of a person or animal. Near UV-C may be relatively unsafe, but may effectively inactivating, especially kill bacteria and viruses. Far UV-C may also be effective in inactivating (killing) bacteria and viruses, but may be (relatively to other UV-C wavelength ranges) (rather) safe. Far-UV light may generate some ozone which may be harmful for human beings and animals. Extreme UV-C may also be effective in inactivating (killing) bacteria and viruses, but may be relatively unsafe. Extreme UV-C may generate ozone which may be undesired when exposed to human beings or animals. In some application ozone may be desired and may contribute to disinfection, but then its shielding from humans and animals may be desired. Hence, in the table "+" for ozone production especially implies that ozone is produced which may be useful for disinfection applications, but may be harmful for humans / animals when they are exposed to it. Hence, in many applications this "+" may actually be undesired while in others, it may be desired. The types of light indicated in above table may in embodiments be used to sanitize air and/or surfaces.

The terms "inactivating" and "killing" with respect to a virus may herein especially refer to damaging the virus in such a way that the virus can no longer infect and/or reproduce in a host cell, i.e., the virus may be (essentially) harmless after inactivation or killing.

Hence, in embodiments, the light may comprise a wavelength in the UV-A range. In further embodiments, the light may comprise a wavelength in the UV-B range. In further embodiments, the light may comprise a wavelength in the Near UV-C range. In further embodiments, the light may comprise a wavelength in the Far UV-C range. In further embodiments, the light may comprise a wavelength in the extreme UV-C range. The Near UV-C, the Far UV-C and the extreme UV-C ranges may herein also collectively be referred to as the UV-C range. Hence, in embodiments, the light may comprise a wavelength in the UV-C range. In other embodiments, the light may comprise violet radiation. Hence, light or radiation described herein may also be indicated as disinfection light.

Prior art solutions may provide Near UV-C radiation based on 254 nm discharge sources, where lamella optics are applied to collimate the Near UV-C radiation in a narrow beam and restrict it to the upper part of rooms since emission at 254 nm is harmful for human skin and eyes. Additional to the potentially harmful emission of Near UV-C, such disinfection devices have significant power loss due to absorption in lamella, hence the radiation generating system efficiency is rather low. There is a need for both efficiency and safety improvement of ultraviolet germicidal radiation generating systems.

The present invention focusses, amongst others, on zinc discharge lamps. Such lamps may especially emit at a wavelength of about 214 nm, which may, as indicated above, be relatively safe and be relatively efficient in reducing e.g. the virus load. The (about) 214 nm peak may have a relatively narrow band with. In addition to the about 214 nm emission, there may be a secondary (weaker) peaks at around 203 nm and 206 nm corresponding to zinc ion lines. Further, there may be emission from resonance lines at about 308 nm and emission of additional lines at 330 nm.

Hence, it is an aspect of the invention to provide an alternative radiation generating system which preferably further at least partly obviates one or more of above-described drawbacks. The present invention may have as object to overcome or ameliorate at least one of the disadvantages of the prior art, or to provide a useful alternative.

Hence, in a first aspect, the invention provides a radiation generating system ("system") which may comprise an optical element assembly ("assembly") and a light generating device. In embodiments, the light generating device may be configured to generate device radiation, which may have one or more wavelengths selected from the wavelength range of 100-380 nm. The light generating device may further comprise an elongated element, which may itself comprise an element length. The elongated element may further comprise one or more radiation emitting faces over at least part of the element length. Especially, the device radiation may emanate from the radiation emitting faces during operation of the light generating device. In embodiments, the optical element assembly may comprise a first hollow reflective element and a second hollow reflective element. In particular, these hollow reflective elements may have a first end and a second end. The hollow reflective elements may further taper from the second end to the first end. The hollow reflective elements may yet further (each) have an optical axis. And especially, (each of) the hollow reflective elements may comprise one or more focal points, which may be configured in a plane perpendicular to the optical axis. In particular embodiments, the first ends of the hollow reflective elements may be directed to each other or may at least partly coincide. Further, the planes (comprising the respective one or more focal points) may not coincide. In embodiments, the elongated element may be configured to at least partly coincide with the optical axes. In (other) embodiments, the elongated element may be configured between the first ends and may be perpendicular to the optical axes. In embodiments, the radiation generating system may be configured to generate system radiation which may comprise at least part of the device light escaping from at least one of the hollow reflective elements. Therefore, in embodiments the invention provides a radiation generating system comprising (i) an optical element assembly, and (ii) a light generating device, wherein: (A) the light generating device is configured to generate device radiation having one or more wavelengths selected from the wavelength range of 100-380 nm; the light generating device comprises an elongated element having an element length (LI), wherein the elongated element comprises one or more radiation emitting faces, over at least part of the element length, from which during operation of the light generating device the device radiation emanates; (B) the optical element assembly comprises a first hollow reflective element and a second hollow reflective element, wherein for the hollow reflective elements applies: (a) the hollow reflective element has a first end and a second end; (b) the hollow reflective element tapers from the second end to the first end; (c) the hollow reflective element has an optical axis (O₁,O₂); and (d) the hollow reflective element comprises one or more focal points (F₁,F₂), configured in a plane (P₁,P₂) perpendicular to the optical axis ; (C) the first ends of the hollow reflective elements are directed to each other or coincide, and the planes (P₁,P₂) do not coincide; (D) the elongated element is (i) configured coinciding with the optical axes or (ii) configured between the first ends and perpendicular to the optical axes ; and (E) the radiation generating system is configured to generate system radiation comprising at least part of the device light.

With the present system, it may be possible to disinfect bacteria more efficiently and/or viruses. The system may generate radiation at shorter wavelengths than 254 nm discharge sources, which may present a lower risk to human skin and eyes. Further, this may allow for the system to comprise more efficient beam shaping optics. Hence, in the present invention, the device radiation may be reflected by hollow reflective elements with focal points on non-coinciding planes, which may allow for beam shaping optics that provide sufficient levels of Far UV-C needed for disinfection to the upper parts of rooms with little power loss due to absorption (by e.g. lamella). Meanwhile, the levels of Far UV-C that may reach the lower parts of rooms would not exceed a level that would be hazardous for human presence. In some embodiments where this would be desirable, the beam shaping optics may be configured to especially irradiate the upper parts of rooms. In particular embodiments, a small percentage of radiation in the Near UV-C bandwidth may escape, which may provide an additional efficiency and an additionally detrimental effect on bacteria and/or viruses.

As indicated above, the invention provides in embodiments a radiation generating system. The radiation generating system at least comprises a light generating device. The radiation generating system may also comprise (or may be functionally coupled to) a control system to control the light generating device, especially its operation. The control system may comprise an electrical ballast.

The radiation generating system may operate in an on-mode or an off-mode. In the former mode, the radiation is provided; in the latter mode the light generating device does not provide radiation. Hence, the light generating device may be configured to provide radiation in the on-mode and be switched off in the off-mode. In embodiments, the radiation source may be an ultraviolet radiation source. In further embodiments, the radiation source may be configured to especially provide radiation with a wavelength of about 214 nm. The term "operational mode" may especially refer to an on-mode.

The term "controlling" and similar terms especially refer at least to determining the behavior or supervising the running of an element. Hence, herein "controlling" and similar terms may e.g. refer to imposing behavior to the element (determining the behavior or supervising the running of an element), etc., such as e.g. measuring, displaying, actuating, opening, shifting, changing temperature, etc.. Beyond that, the term "controlling" and similar terms may additionally include monitoring. Hence, the term "controlling" and similar terms may include imposing behavior on an element and also imposing behavior on an element and monitoring the element. The controlling of the element can be done with a control system, which may also be indicated as "controller". The control system and the element may thus at least temporarily, or permanently, functionally be coupled. The element may comprise the control system. In embodiments, the control system and element may not be physically coupled. Control can be done via wired and/or wireless control. The term "control system" may also refer to a plurality of different control systems, which especially are functionally coupled, and of which e.g. one control system may be a master control system and one or more others may be slave control systems. A control system may comprise or may be functionally coupled to a user interface.

The control system may also be configured to receive and execute instructions form a remote control. In embodiments, the control system may be controlled via an App on a device, such as a portable device, like a Smartphone or I-phone, a tablet, etc.. The device is thus not necessarily coupled to the lighting system, but may be (temporarily) functionally coupled to the lighting system.

Hence, in embodiments the control system may (also) be configured to be controlled by an App on a remote device. In such embodiments the control system of the lighting system may be a slave control system or control in a slave mode. For instance, the lighting system may be identifiable with a code, especially a unique code for the respective lighting system. The control system of the lighting system may be configured to be controlled by an external control system which has access to the lighting system on the basis of knowledge (input by a user interface of with an optical sensor (e.g. QR code reader) of the (unique) code. The lighting system may also comprise means for communicating with other systems or devices, such as on the basis of Bluetooth, Thread, WIFI, LiFi, ZigBee, BLE or WiMAX, or another wireless technology.

The system, or apparatus, or device may execute an action in a "mode" or "operation mode" or "mode of operation" or "operational mode". The term "operational mode may also be indicated as "controlling mode". Likewise, in a method an action or stage, or step may be executed in a "mode" or "operation mode" or "mode of operation" or "operational mode". This does not exclude that the system, or apparatus, or device may also be adapted for providing another controlling mode, or a plurality of other controlling modes. Likewise, this may not exclude that before executing the mode and/or after executing the mode one or more other modes may be executed.

However, in embodiments a control system may be available, that is adapted to provide at least the controlling mode. Would other modes be available, the choice of such modes may especially be executed via a user interface, though other options, like executing a mode in dependence of a sensor signal or a (time) scheme, may also be possible. The operation mode may in embodiments also refer to a system, or apparatus, or device, that can only operate in a single operation mode (i.e. "on", without further tunability).

Hence, in embodiments, the control system may control in dependence of one or more of an input signal of a user interface, a sensor signal (of a sensor), and a timer. The term "timer" may refer to a clock and/or a predetermined time scheme.

The term "radiation source" may in principle relate to any radiation source known in the art. It may be a conventional (tungsten) light bulb, a low pressure mercury lamp, a high pressure mercury lamp, a fluorescent lamp, a LED (light emissive diode). In a specific embodiment, the radiation source comprises a solid state LED radiation source (such as a LED or laser diode (or "diode laser")). The term "radiation source" may also relate to a plurality of radiation sources, such as 2-200 (solid state) LED radiation sources. Hence, the term LED may also refer to a plurality of LEDs. Further, the term "radiation source" may in embodiments also refer to a so-called chips-on-board (COB) radiation source. The term "COB" especially refers to LED chips in the form of a semiconductor chip that is neither encased nor connected but directly mounted onto a substrate, such as a PCB. Hence, a plurality of light emitting semiconductor radiation source may be configured on the same substrate. In embodiments, a COB is a multi LED chip configured together as a single radiation module.

The radiation source may have a radiation escape surface. Referring to conventional radiation sources such as light bulbs or fluorescent lamps, it may be outer surface of the glass or quartz envelope. For LED's it may for instance be the LED die, or when a resin is applied to the LED die, the outer surface of the resin. In principle, it may also be the terminal end of a fiber. The term escape surface especially relates to that part of the radiation source, where the radiation actually leaves or escapes from the radiation source. The radiation source is configured to provide a beam of radiation. This beam of radiation (thus) escapes from the radiation exit surface of the radiation source.

The term "radiation source" may refer to a semiconductor light-emitting device, such as a light emitting diode (LEDs), a resonant cavity light emitting diode (RCLED), a vertical cavity laser diode (VCSELs), an edge emitting laser, etc... The term "radiation source" may also refer to an organic light-emitting diode (OLED), such as a passive-matrix (PMOLED) or an active-matrix (AMOLED). In a specific embodiment, the radiation source comprises a solid-state radiation source (such as a LED or laser diode). In an embodiment, the radiation source comprises a LED (light emitting diode). The terms "radiation source" or "solid state radiation source" may also refer to a superluminescent diode (SLED).

The term LED may also refer to a plurality of LEDs.

The term "radiation source" may also relate to a plurality of (essentially identical (or different)) radiation sources, such as 2-2000 solid state radiation sources. In embodiments, the radiation source may comprise one or more micro-optical elements (array of micro lenses) downstream of a single solid-state radiation source, such as a LED, or downstream of a plurality of solid-state radiation sources (i.e. e.g. shared by multiple LEDs). In embodiments, the radiation source may comprise a LED with on-chip optics. In embodiments, the radiation source comprises a pixelated single LEDs (with or without optics) (offering in embodiments on-chip beam steering).

In embodiments, the radiation source may be configured to provide primary radiation, which is used as such, such as e.g. a blue radiation source, like a blue LED, or a green radiation source, such as a green LED, and a red radiation source, such as a red LED. Such LEDs, which may not comprise a luminescent material ("phosphor") may be indicated as direct color LEDs.

In other embodiments, however, the radiation source may be configured to provide primary radiation and part of the primary radiation is converted into secondary radiation. Secondary radiation may be based on conversion by a luminescent material. The secondary radiation may therefore also be indicated as luminescent material radiation. The luminescent material may in embodiments be comprised by the radiation source, such as a LED with a luminescent material layer or dome comprising luminescent material. Such LEDs may be indicated as phosphor converted LEDs or PC LEDs (phosphor converted LEDs).

In other embodiments, the luminescent material may be configured at some distance ("remote") from the radiation source, such as a LED with a luminescent material layer not in physical contact with a die of the LED.

The radiation source may especially be configured to generate radiation source radiation having an optical axis (O), (a beam shape,) and a spectral power distribution. The radiation source radiation may in embodiments comprise one or more bands, having band widths as known for lasers

The term "radiation source" may (thus) refer to a radiation generating element as such, like e.g. a solid state radiation source, or e.g. to a package of the radiation generating element, such as a solid state radiation source, and one or more of a luminescent material comprising element and (other) optics, like a lens, a collimator. A radiation converter element ("converter element" or "converter") may comprise a luminescent material comprising element. For instance, a solid state radiation source as such, like a blue LED, is a radiation source. A combination of a solid state radiation source (as radiation generating element) and a radiation converter element, such as a blue LED and a radiation converter element, optically coupled to the solid state radiation source, may also be a radiation source (but may also be indicated as light generating device). Hence, a white LED is a radiation source (but may e.g. also be indicated as (white) light generating device).

In embodiments, the light generating device may comprise a luminescent material. In embodiments, the light generating device may comprise a PC LED. In other embodiments, the light generating device may comprise a direct LED (i.e. no phosphor). In embodiments, the light generating device may comprise a laser device, like a laser diode. In embodiments, the light generating device may comprise a superluminescent diode. Hence, in specific embodiments, the radiation source may be selected from the group of laser diodes and superluminescent diodes. In other embodiments, the radiation source may comprise an LED.

The term "radiation source" herein may also refer to a radiation source comprising a solid state radiation source, such as an LED or a laser diode or a superluminescent diode.

The "term radiation source" may (thus) in embodiments also refer to a radiation source that is (also) based on conversion of radiation, such as a radiation source in combination with a luminescent converter material. Hence, the term "radiation source" may also refer to a combination of a LED with a luminescent material configured to convert at least part of the LED radiation, or to a combination of a (diode) laser with a luminescent material configured to convert at least part of the (diode) laser radiation.

In embodiments, the term "radiation source" may also refer to a combination of a radiation source, like a LED, and an optical filter, which may change the spectral power distribution of the radiation generated by the radiation source. Especially, the "term light generating device" may be used to address a radiation source and further (optical components), like an optical filter and/or a beam shaping element, etc.

The phrases "different radiation sources" or "a plurality of different radiation sources", and similar phrases, may in embodiments refer to a plurality of solid-state radiation sources selected from at least two different bins. Likewise, the phrases "identical radiation sources" or "a plurality of same radiation sources", and similar phrases, may in embodiments refer to a plurality of solid-state radiation sources selected from the same bin.

The term "solid state radiation source", or "solid state material radiation source", and similar terms, may especially refer to semiconductor radiation sources, such as a light emitting diode (LED), a diode laser, or a superluminescent diode. Especially, the term "radiation source" herein may refer to a light emitting diode (LED), a diode laser, or a superluminescent diode. For instance, the radiation source may comprise one or more LEDs.

In embodiments, the light generating device may comprise a fluorescent tube, a LED filament, or a discharge vessel based light generating device. Especially, the invention is herein explained in relation to the latter.

Especially, the light generating device may comprise a discharge source based on one or more of zinc, mercury, cadmium, xenon, and krypton. Here below, the invention is especially described in relation to a zinc based discharge light generating device. However, the invention is not limited to such embodiments and may also related to e.g. a Kr-Cl based lamp, or a mercury based lamp, etc., see also below.

The radiation generating system may comprise a mercury free discharge lamp. In specific embodiments, the radiation generating system may further comprise (ii) a lamp control system. Especially, the discharge lamp may comprise a discharge vessel, and electrodes. In embodiments, the discharge vessel may enclose at room temperature (a) one or more pieces of metal, and (b) a gas composition. In specific embodiments, the one or more pieces of metal may consist for at least 90 mol% of zinc, such as especially at least 99.99 mol% zinc. In specific embodiments, the gas composition may have a pressure selected from the range of 5-300 mbar. Yet, the gas composition may consist for at least 85 mol%, more especially at least 90 mol%, such as yet more especially at least 95 mol% of noble gas. Especially, the radiation generating system may be configured to generate system radiation having spectral power in the wavelength range of 200-230 nm. Hence, in specific embodiments the invention provides a radiation generating system comprising (i) a mercury free discharge lamp, wherein the discharge lamp comprises a discharge vessel, and electrodes, wherein the discharge vessel encloses at room temperature (a) one or more pieces of metal, and (b) a gas composition; wherein: (A) the one or more pieces of metal consist for at least 90 mol% of zinc, such as especially at least 99.99 mol% zinc; (B) the gas composition has a pressure selected from the range of 5-300 mbar, and consists for at least 85 mol%, yet especially at least about 95 mol% of noble gas; and (C) the radiation generating system is configured to generate system radiation having spectral power in the wavelength range of 200-230 nm.

Especially, in embodiments the discharge lamp (more especially the discharge vessel) may have an (essentially) axial design. Therefore, in embodiments the discharge vessel may have a cylindrical design. The electrodes may be configured external of the cylinder (see further also below). As indicated above, in embodiments the discharge vessel may have a cylindrical design. However, other shapes may also be possible.

Further, in embodiments the discharge vessel may at least partly be defined by a discharge vessel wall. Such discharge vessel wall may especially comprise quartz. Alternatively, the discharge vessel wall may comprise e.g. CaF₂, BaF₂, MgF₂, or Al₂O₃ (sapphire or PCA). Especially, however, the discharge vessel may be of quartz.

As indicated above, the radiation generating system comprises a mercury free discharge lamp. The term "mercury free discharge lamp" may especially indicate that mercury is not deliberately added to the discharge vessel (as filling component). Further, mercury is essentially not necessary for starting and/or propagation of the discharge. Relative to the amount of zinc (Zn) added as filling in the form or one or more pieces comprising zinc, the amount of mercury may be less than 20 ppm relative to zinc, such as less than about 10 ppm. In specific embodiments, the mercury amount in terms of dosed amounts may be less than 20 ppm of the zinc dose to ensure that all mercury, if any, in the gas phase is at least a factor of 10 (and preferably more, such as at least a factor of 20, like at least a factor of 50) lower than the amount of zinc in the gas phase at the operating temperature of the lamp.

Further, the radiation generating system may comprise a lamp control system. The lamp control system may especially be configured to start the discharge and maintain the discharge. Hence, the lamp control system may e.g. comprise ballast. Such lamp control system may essentially be the same as used for regular fluorescent lamps or mercury based UV-C lamps. In embodiment, the control system may comprise a driver. The driver may comprise an inductive coil, or a combination of an inductive coil and a capacitor. The driver may operate at the mains frequency of 50/60 Hz, or the driver may be a full electronic driver operating at a frequency of e.g. 10-100 kHz. However, other embodiments may also be possible. The potential difference between the electrodes may in embodiments e.g. be at least about 15 V, such as at least about 20 V. Further, the potential difference between the electrodes may e.g. be at maximum about 500 V, such as at maximum about 300 V. In specific embodiments, the potential difference between the electrodes may e.g. be selected from the range of about 50-100 V. However, other potential differences may also be possible.

Hence, especially the light generating device may comprise a low pressure lamp comprising a zinc based discharge source. The strongest resonance line of zinc is located at about 214 nm and the next strong line is present at 308 nm. In embodiments, an almost monochromatic source of Far UV-C may be produced at around 214 nm, which may depend upon the zinc pressure, which is determined by the vapor pressure of zinc above the coldest spot in the light generating device. Especially, in embodiments, the zinc pressure may be high enough to have any emission of the 214 nm resonance line, but may be low enough to have a non-equilibrium discharge with a favorable electron energy distribution function. Further, additional measures may be implemented to reach a cold spot temperature inside the discharge vessel to achieve the desired zinc vapor pressure, e.g. high enough power density, thermal insulation, or auxiliary heating systems.

Hence, the operation conditions may be chosen such that the temperature of the cold spot is not lower than a threshold value, like at least about 200 °C, such as at least about 250 °C. Therefore, in embodiments the lamp control system may be configured to maintain a cold spot temperature during operation at a temperature of at least 250°C. Hence, during operation the cold spot temperature may be at least 250°C. More especially, the cold spot temperature may be at least about 295°, especially for a good performance, more especially at least about 300 °C, such as at least about 320 °C. A too high temperature of the cold spot may lead to a less efficient discharge. Hence, the cold spot temperature may be especially not larger than about 400 °C, such as especially not larger than about 360 °C. Therefore, in embodiments the lamp control system is configured to maintain a cold spot temperature during operation at a temperature of at least 295 °C and at maximum 360 °C, such as in the range of 295-400 °C, like especially in the range of 300-360 °C. Especially, the cold spot temperature may be selected from the range of 320-360 °C, like in more specific embodiments in the range of about 325-350 °C. Hence, during operation the cold spot temperature may be at least 295 °C and at maximum 360 °C.

In embodiments, the radiation generating system may comprise an auxiliary heating system. The auxiliary heating system may especially be configured to heat part of one or both ends of the discharge vessel and/or to heat one or both electrodes. The auxiliary heating system may be comprised by the lamp control system. Alternatively or additionally, the auxiliary heating system may be configured to direct a current through the electrodes, using the radiation heat from the electrodes to heat up the wall area around the electrode region (where a cold spot could form).

During operation, part of the zinc may get into the gaseous phase. This may lead to a zinc partial pressure. In embodiments, the zinc partial pressure may at least be about 0.0005 mbar. Further, in embodiments the zinc partial pressure may at maximum be about 150 mbar. Especially, in embodiments the lamp control system is configured to maintain a zinc pressure in the discharge vessel during operation in the range of 0.001-100 mbar. Hence, during operation the zinc pressure in the discharge vessel may be in the range of 0.001-100 mbar. Especially, in embodiments the zinc partial pressure may be selected from the range of about 0.001-0.5 mbar, such as selected from the range of about 0.005-0.2 mbar, which may be considered a low-pressure application. Therefore, in specific embodiments the lamp control system is configured to maintain a zinc pressure in the discharge vessel during operation in the range of 0.005-0.2 mbar. In yet more specific embodiments, during operation the zinc pressure in the discharge vessel during operation in the range of 0.005-0.05 mbar, such as in the range of 0.01-0.02 mbar.

As indicated above a partial pressure *P1* of zinc during operation may be selected from the range of 0.001-100 mbar, especially during an operation wherein the cold spot temperature is selected from the range of 295-400 °C, like especially in the range of 300-360 °C, such as 320-360 °C, like in more specific embodiments in the range of about 325-350 °C.

The gas composition may especially be devoid of halogen comprising gas. Nevertheless, the gas composition may comprise a halogen comprising gas, though especially the content may be kept low. Hence, in embodiments the gas composition may be chosen such, that the partial pressure (P2) of the halogen comprising gas may be equal to or smaller than 0.2^{∗}*P1*, such as equal to or smaller than 0.1^{∗}*P1* (during an operation wherein the cold spot temperature is selected from the range of 295-400 °C, like especially in the range of 300-360 °C, such as 320-360 °C, like in more specific embodiments in the range of about 325-350 °C). Here, the partial pressure of the halogen comprising gas may refer to the partial pressure defined by all available halogen comprising gases, which may especially be one or mor of the above mentioned halogen comprising gas.

In embodiments, the wall power density may be selected such that generation and maintenance of the discharge is facilitated. Especially, the wall power density may be selected from the range of about 0.15-5 W/cm², more especially about 0.2-2.5 W/cm², such as about 0.3-2 W/cm². Hence, in specific embodiments the lamp control system may be configured to maintain a wall power density Pw selected from the range of 0.3-2 W/cm², wherein the wall power density P_{W} is defined as P_{W}=P/(π^{∗}D₂^{∗}d_{EL}), wherein P is the dissipated power, D₂ is the external diameter of the discharge vessel, and d_{EL} is the distance between the two electrodes configured at opposite ends of the discharge vessel. In embodiments, wall power density Pw selected from the range of 0.5-1.8 W/cm². Pw is the dissipated lamp power, given by the lamp voltage times the lamp current. Hence, the wall power density P_{W} may be selected from the range of 0.3-2 W/cm².

As indicated above, the gas composition may essentially consist of noble gas. More especially, in embodiments the gas composition may comprise one or more of Ne, Ar, Kr, and Xe. Especially, the presence of argon (Ar) may be desirable. Alternatively or additionally, it seems desirable that at least two different noble gasses are comprised by the gas composition. Therefore, in specific embodiments the gas composition may comprise Ar and one or more of Ne and Kr. Alternatively, in embodiments the gas composition may comprise Ne and one or more of Ar and Kr. A combination of Ar and Ne seems to lead to the best results in terms of creating the discharge and maintaining the discharge. In embodiments, the gas composition may comprise 0.005-5 vol.% Ar, more especially 0.01-2 vol.% Ar. In other embodiments, the gas composition essentially consists of argon. The latter embodiment may e.g. be desirable in view of lifetime.

As indicated above, for achieving desirable temperature, it may also be an option to have part of the discharge vessel enclosed in a reflective material and/or thermally insulating material. Therefore, in embodiments at at least one of the opposite ends of the discharge vessel, a part of the discharge vessel may be enclosed by a material having one or more of a radiation reflective and thermally insulating properties. In embodiments, for achieving desirable temperature, it may also be an option to have part of the discharge vessel enclosed in material having a relatively low emissivity (ε). Hence, in embodiments at at least one of the opposite ends of the discharge vessel, a part of the discharge vessel may be enclosed by a material having an emissivity of at maximum 0.5 at 300 °C, such as at maximum about 0.1. For instance, in embodiments one or both ends may comprise a coating and/or may be wrapped in a material which is reflective for the radiation of the zinc discharge, and/or is thermally insulating, and/or has a low emissivity. For instance, in embodiment the material may be an aluminum, silver, copper, or gold coating, or aluminum foil. Combinations of two or more of such options may also be applied.

In further embodiments, the light generating device may also comprise an excimer lamp. Especially, the excimer lamp may be a dielectric barrier discharge (DBD) lamp. Dielectric barrier discharges are a mercury free source of UV radiation. The use of halogens like bromine and chlorine may enable the generation of UV in the range of about 200-230 nm. As indicated above, this radiation can inactivate harmful viruses by destroying their DNA/RNA, and/or inactivate other parts of the virus proteins. Their wavelength is substantially long enough to prevent the generation of substantial amounts of ozone and substantially short enough not the reach the living skin cells or the cornea of humans possibly present when the UV source is operational.

Hence, in embodiments the light generating device may be configured to generate device radiation having one or more wavelengths selected from the wavelength range of 100-380 nm. The light generating device may generate UV-C device radiation having one or more wavelengths selected from the range of 100-280 nm. The light generating device may especially generate Far UV-C device radiation having one or more wavelengths selected from the range of 190-230 nm. In particular, the light generating device may generate Far UV-C device radiation from around 214 nm.

Hence, the generation of Far UV-C device radiation may provide safer disinfection via irradiation compared to light generating systems which generate Near UV-C device radiation.

The light generating device may comprise an elongated element having an element length L1. The elongated element may especially be cylindrical in shape. The elongated element may also be polygonal in shape. Yet further shapes may also be possible.

Hence, the shape of the elongated element may depend on the desired beam optics and radiation properties of embodiments.

The elongated element may comprise one or more radiation emitting faces over at least part of the element length L1. The radiation emitting face may comprise a surface that allows transmission of device radiation. In embodiments, the radiation emitting face may be curved or flat. The radiation emitting face may comprise materials that allow transmission of device radiation, such as clear glass or quartz material, though other materials may also be possible, dependent upon the light generating device. The radiation emitting faces cover at least part of the element length L1 of the light generating device. During operation of the light generating device, the device radiation emanates from the radiation emitting face. Hence, the elongated element may also be indicated as "light generating element" or "light source".

Hence, when device radiation has been generated by the discharge source of the light generating device, it may first emanate through the radiation emitting face. The shape and material of the radiation emitting face depends on properties of the elongated element, in turn depending on the desired beam optics and radiation properties of embodiments. In the case of a discharge vessel, at least part of the outer surface of the vessel wall may define the radiation emitting face.

Hence, in embodiments essentially an entire outer face of the discharge vessel, which e.g. be a quartz discharge vessel may be the radiation emitting face. However, would e.g. a LED strip be applied, there may be a plurality of radiation emitting faces.

Likewise, in embodiments the discharge vessel is at least transmissive for radiation in the wavelength range of 200-230 nm. Hence, radiation having a wavelength selected from the wavelength range of 200-230 nm may be transmitted through the wall of the discharge vessel. The transmission (for radiation having a wavelength selected from the wavelength range of 200-230 nm) may e.g. be at least 30%, such as at least 50%.

Further, in embodiments the discharge vessel may at least partly be defined by a discharge vessel wall. Such discharge vessel wall may especially comprise quartz. Alternatively, the discharge vessel wall may comprise e.g. CaF₂, BaF₂, MgF₂ Especially, however, the discharge vessel may be of quartz.

In embodiments, the discharge vessel may have an outer diameter D₂ selected from the range of 8-38 mm. Further, the discharge vessel may have a wall thickness d₁ selected from the range of 0.4-2.0 mm, like at least about 0.7 mm, such as up to about 1.5 mm, like 0.5-1.5 mm, such s 0.7-1.2 mm. Especially, in embodiments the discharge vessel may have an outer diameter D₂ selected from the range of about 11-26 mm. The discharge vessel may have an inner diameter D₁ selected from the range of 4-36 mm.

Hence, especially in embodiments the discharge vessel may have an inner diameter D₁ selected from the range of 4-36 mm, the discharge vessel may have a vessel wall having a wall thickness (d₅) selected from the range of 0.4-2.0 mm, wherein the electrodes may be configured within the discharge vessel

The optical element assembly may comprise a first hollow reflective element and a second hollow reflective element. The hollow reflective elements may both be from a material reflective to the device radiation, such as aluminum, silver, copper, or gold. Alternatively or additionally, the hollow reflective elements may have a reflective coating, such as aluminum, silver, copper, or gold coating.

Herein, the invention is mainly described in relation to hollow reflective elements that may essentially be the same (but configured at opposite side of a mirror plane). Especially, there may be a mirror plane between the planes comprising the one or more focal points. However, the hollow reflective elements are not necessarily the same. Here below, the hollow reflective elements are described in general, and the embodiments described may especially apply for both hollow reflective elements. However, in other embodiments different hollow reflective elements may be comprised by the assembly.

The hollow reflective element may be defined by a first end and a second end. The hollow reflective element may be further defined as tapering from the second end to the first end. The hollow reflective element may further have a curve or flat surface, and may comprise various shapes in embodiments.

The first end may be a first opening and the second end may be a second opening, as the reflective elements are hollow. The first opening may in embodiments be circular or may in other embodiments be rectangular, though other shapes are herein not excluded. The second opening may in embodiments be circular or may in other embodiments be rectangular, though other shapes are herein not excluded. In general, though not exclusively, the first opening and the second opening may essentially have the same shape.

Hence, the hollow reflective element may further reflect the device radiation that has emanated from the radiation emitting face. The shape of the hollow reflective element may depend on the desired beam.

The hollow reflective element may comprise an optical axis. Especially, the term "optical axis" may be defined as an imaginary line that defines the path along which light propagates through a system starting from the light generating element, here especially the light generating device. Especially, the optical axis may coincide with the direction of the light with the highest radiant flux.

The optical axes of the hollow reflective elements may be colinear or may coincide.

The hollow reflective element may comprise one or more focal points . The focal point may be defined as the point at which the device radiation beams converge, would the device radiation propagate in an opposite direction than during operation of the system, i.e. in a direction from the second end to the first end (instead of during operation from about the first end to the second end).

Each of the two hollow reflective elements may have one or more focal points. For a single hollow reflective element, there may be a single focal point, but there may also be more than one focal points, like a line (comprising two or more focal points), such as may be in the case of an elongated hollow reflective element. The focal point may be on the optical axis, but that is not necessarily the case. When there is a focal line, such focal line may especially be configured perpendicular to the optical axis.

For the sake of definition, a virtual plane is used, wherein the focal point may be configured, and which virtual plane is configured perpendicular to the optical axis. Hence, the focal point may be configured in a plane (P₁,P₂) (or virtual plane) perpendicular to the optical axis . The plane may comprise a single point, line, circle, or geometric plane.

These virtual planes for the first hollow reflective element and second hollow reflective element may especially be configured parallel, though this is not necessarily the case.

Further, these virtual planes are especially be defined to be comprised by the hollow reflective element. Hence, these planes also do not have a secant line. However, external from the hollow reflective elements, the virtual planes may have a secant line. Especially, however the virtual planes comprise the foci of the respective hollow elements do not have a secant line and are configured parallel. Hence, in embodiments the planes (P₁,P₂) may not coincide, and are especially configured parallel.

Hence, the optical axis, focal points, and planes of the hollow reflective elements may together define the beam shaping optics of the radiation generating system. In embodiments, these properties may be adjusted to achieve the desired beam shaping optics, allowing for optimal UV radiation, especially UV-C irradiation of the upper parts of rooms while facilitating non-hazardous of less hazardous UV radiation exposure to the lower parts of rooms (see also below).

In specific embodiments, the first ends of the hollow reflective elements may be directed to each other. In further specific embodiments, the first ends of the hollow reflective elements may coincide. In such embodiments, the first end openings of the hollow reflective elements may coincide. Hence, the seconds end may be relatively remote from each other and the first ends are relatively close to each other, or may even coincide.

Hence, the beam shaping optics of the radiation generating system may be configured via the first ends of the hollow reflective elements such that their planes (and consequently, focal points) do not coincide. This may in embodiments facilitate for efficient beam shaping optics and consequently lower energy requirements.

Especially, there may be two relevant embodiments of the elongated element.

In embodiments, the elongated element may be configured at least partly coinciding with the optical axis . Especially, in embodiments an axis of elongation of the elongated element may essentially coincide with the optical axes of the hollow reflective elements. Hence, the phrase "the elongated element may be configured at least partly coinciding with the optical axis " may especially indicate that an axis of elongation of the elongated element and the optical axes of the hollow reflective elements may essentially coincide.

Alternatively, in embodiments, the elongated element may be configured between the first ends and perpendicular to the optical axis . Further, in such embodiments the optical axes of the hollow reflective elements may be configured perpendicular to an axis of elongation of the elongated element.

Hence, in the former embodiments the elongated element may protrude into the hollow reflective elements, whereas in the latter embodiments the elongated element may be configured between the two first ends and will not (substantially) protrude into the hollow reflective elements.

Hence, in embodiments, the configuration may provide for beam shaping optics that result in radiation beams that are narrow in one direction and broad in the orthogonal direction. In further embodiments, the configuration may provide for beam shaping optics that result in radiation beams that may substantially be revolved around their optical axis . Yet further embodiments may facilitate other radiation beam shapes.

The radiation generating system may be configured to generate system radiation comprising at least part of the device light escaping from at least one of the hollow reflective elements.

In specific embodiments, at least 25% of the spectral power of the system radiation in the wavelength range of 200-500 nm may be provided by spectral power in the wavelength range of 200-230 nm. More especially, the spectral power in the 200-230 nm wavelength range may be in the range of at least 40% of the total spectral power in the 200-500 nm wavelength range. For instance, the spectral power in the 200-230 nm wavelength range may be in the range of 25-60% of the total spectral power in the 200-500 nm wavelength range. Yet, in embodiments the spectral power in the 200-230 nm wavelength range may be in the range of at least 50% of the total spectral power in the 200-300 nm wavelength range, or even higher.

In specific embodiments, the elongated element may comprise a tubular element. In further embodiments, the tubular element itself may comprise an external cylindrical wall with an external diameter (D₂). In such embodiments, the external cylindrical wall may comprise the radiation emitting face(s).

Hence, in specific embodiments, the arrangement of the elongated element and the radiation emitting faces may be defined by a tubular element comprising a cylindrical wall. A cylindrical shape may facilitate device radiation emanation that may be reflected by the hollow reflective elements in desired beam shaping optics.

In specific embodiments, the optical element assembly may be elongated. In such embodiments, the optical element assembly may be further defined by an axis of elongation (A₁) and a second length (L2). Further, in such embodiments, the elongated element and the axis of elongation (A₁) may be in parallel configuration. In specific embodiments, 0.9≤L2/L1≤5. For instance, 0.9≤L2/L1≤4. In specific embodiments, 0.9≤L2/L1≤1.5. Especially, in embodiments 1.0≤L2/L1≤1.2. Hence, an optical element assembly defined by an axis of elongation (A₁) and a second length (L2) may facilitate desired beam shaping optics. Especially, this may facilitate such beam shaping optics which result in beams that are narrow in one direction and broad in the orthogonal direction.

In embodiments, the hollow reflective elements may have a parabola-like cross-sectional shape. More especially, the hollow reflective elements may have a parabolic cross-sectional shape. In embodiments, the hollow reflective elements may have compound parabolic cross-sectional shape. A perpendicular cross-sectional shape may e.g. be rectangular. Especially, in such embodiments the elongated elements may be configured between the first ends (411,421) and perpendicular to the optical axes .

In embodiments, the planes (P₁,P₂) may have a mutual distance (d₁). The mutual distance may e.g. be selected from the range of about 1-100 mm, more especially about 1-50 mm. In embodiments, the mutual distance (d₁) may be defined as 0.8≤d₁/D₂≤1.25. Especially, the mutual distance (d₁) may be defined as 0.9≤d₁/D₂≤1.1. This may especially be the case when the elongated elements may be configured between the first ends (411,421) and perpendicular to the optical axes .

Hence, the shape of the hollow reflective elements and the mutual distance (d1) between the planes (P₁,P₂) may be adjusted in embodiments to provide the desired beam shaping optics, especially in such embodiments that are configured for generating beams that are narrow in one direction and broad in the orthogonal direction. Further, as the size of the parabola-like or parabolic shape increases, it may decrease the convection air speed, which may provide a benefit for the thermal insulation of the optical element assembly.

As indicated above, in (alternative) embodiments the elongated element (150) may be configured at least partly coinciding with the optical axes .

In specific embodiments, the hollow reflective elements may have two orthogonal parabola-like cross-sectional shapes. Especially, the hollow reflective elements may have two orthogonal parabolic cross-sectional shapes. In embodiments, the hollow reflective elements may have two orthogonal compound parabolic cross-sectional shapes. Especially, this may facilitate such beam shaping optics which result in beams that are narrow in one direction and broad in the orthogonal direction.

As indicated above, in embodiments, the planes (P₁,P₂) may have a mutual distance (d₁). The mutual distance may e.g. be selected from the range of about 1-100 mm, more especially about 1-50 mm.

In embodiments, a middle (MP) between the planes (P₁,P₂) and a middle (ME) of the elongated element may be defined. In such embodiments, these middles (MP,ME) may be configured within distance of each other of at maximum 0.1^{∗}d₁, wherein 1.5≤L1/d₁≤2.5. Especially, these middles (MP,ME) may be configured within distance of each other of at maximum 0.1^{∗}d₁, wherein 1.9≤L1/d₁≤2.1. Especially, these middles (MP,ME) may be configured within distance of each other of at maximum 0.05^{∗}d₁, wherein 1.9≤L1/d₁≤2.1. Especially, this may apply in embodiments wherein the elongated element (150) may be configured at least partly coinciding with the optical axes .

Hence, the shape of the hollow reflective elements and the mutual distance (d₁) between the planes (P₁,P₂) may be adjusted in embodiments to provide the desired beam shaping optics, especially in such embodiments which are configured to generate radiation beams that are revolved around their optical axis . Such embodiments may also facilitate improved thermal insulation of the discharge source. Further, as the size of the parabola-like or parabolic shape increases, it may decrease the convection air speed, which may provide a benefit for the thermal insulation of the optical element assembly.

In such embodiments with beam shaping optics as described above, the mentioned parabola-like cross-sectional shapes of the hollow reflective elements may have the shape of a cross-section of a compound parabolic reflector. Especially, in such embodiments with parabolic cross-sectional shapes of the hollow reflective elements, these may have the shape of a cross-section of a compound parabolic reflector.

Hence, in embodiments, the hollow reflective elements may have the shape of a cross-section of a compound parabolic reflector which may provide the desired beam shaping optics.

In embodiments, the hollow reflective elements may be comprised by one or more thermally conductive bodies. The thermally conductive bodies may comprise thermally conductive materials, such as metals. A thermally conductive material may especially have a thermal conductivity of at least about 20 W/(m^{∗}K), like at least about 30 W/(m^{∗}K), such as at least about 100 W/(m^{∗}K), like especially at least about 200 W/(m^{∗}K). In yet further specific embodiments, a thermally conductive material may especially have a thermal conductivity of at least about 10 W/(m^{∗}K). In embodiments, the thermally conductive material may comprise one or more of copper, aluminum, silver, gold, silicon carbide, aluminum nitride, boron nitride, aluminum silicon carbide, beryllium oxide, a silicon carbide composite, aluminum silicon carbide, a copper tungsten alloy, a copper molybdenum carbide, carbon, diamond, and graphite. Alternatively, or additionally, the thermally conductive material may comprise or consist of aluminum oxide.

The assembly may in embodiments be thermally coupled, such as in physical contact with one or more of a heat sink, a heat spreader, and a two-phase cooling device.

Hence, the hollow reflective elements may be provided with thermal conductivity, which may facilitate the retention of sufficiently high temperatures which are required for the discharge source to provide device radiation.

In embodiments, the light generating device and at least one of the hollow reflective elements may be configured to generate a beam of system radiation with at least one cross-section perpendicular to the optical axis with a full width at half maximum of at maximum 35°. Especially, the configuration may generate a beam of system radiation having a full width at half maximum of at maximum, 30°, and especially as at least about 10°. However, other values may also be possible.

The optional full width half maximum conditions as indicated above may apply for one cross-section of the beam or for two orthogonal cross-sections, especially dependent upon the elongated element is (i) configured at least partly coinciding with the optical axes or (ii) configured between the first ends and perpendicular to the optical axes. When the elongated element is configured at least partly coinciding with the optical axes , in embodiments two orthogonal cross-sections of the beam of system radiation may be at maximum 35°. When the elongated element is configured between the first ends and perpendicular to the optical axes , a single cross-section of the beam of system radiation may be at maximum 35°.

Further, the invention provides in an aspect a method for treating a gas or a surface with the radiation generating system as e.g. described in embodiments above. Especially, the gas or the surface may be external from the radiation generating system. Hence, in embodiments the method may comprise providing the system radiation with the radiation generating system to the gas or the surface external to the radiation generating system. Especially, the method may comprise a configuration in which the upper part of a room is irradiated more than the lower part of rooms.

In embodiments, the method may comprise exposing air (external from the system) to the radiation from the system. Hence, in specific embodiments, the method for treating air may comprise: exposing air to the radiation from the system. In this way, the method may provide one or more of disinfection of pathogens, removal of particles and dust, and removal of odors. Especially, the treatment of the air may comprise disinfection of (the) air. In embodiments, the method may comprise exposing a surface to the radiation from the system. The surface may be selected from a desk, a floor, a wall, a kitchen counter, a door handle, a tap, a handrail, a control panel, etc. The embodiments described above in relation to the system of the present invention, may also apply for the method of the invention. The radiation may be provided in a space, e.g. in the method for treating a gas or a surface (available in the space).

The term "space" may for instance relate to a (part of) hospitality area, such as a restaurant, a hotel, a clinic, or a hospital, etc.. The term "space" may also relate to (a part of) an office, a department store, a warehouse, a cinema, a church, a theatre, a library, etc. However, the term "space" may also relate to (a part of) a working space in a vehicle, such as a cabin of a truck, a cabin of an airplane, a cabin of a vessel (ship), a cabin of a car, a cabin of a crane, a cabin of an engineering vehicle like a tractor, etc.. The term "space" may also relate to (a part of) a working space, such as an office, a (production) plant, a power plant (like a nuclear power plant, a gas power plant, a coal power plant, etc.), etc. For instance, the term "space" may also relate to a control room, a security room, etc. Especially, the term "space" may herein refer to an indoor space. In yet other embodiments, the term "space" may also relate to a toilet room or bathroom. In yet other embodiments, the term "space" may also relate to an elevator. In embodiments, the term "space" may also refer to a conference room, a school room, an indoor hallway, an indoor corridor, an indoor space in an elderly home, an indoor space in a nursing home, etc. In embodiments, the term "space" may refer to an indoor sport space, like a gym, a gymnastics hall, in indoor ball sport space, a ballet room, a swimming pool, a changing room, etc. In embodiments, the term "space" may refer to an (indoor) bar, an (indoor) disco, etc.

In embodiments, the method may comprise configuring the radiation generating system in a space comprising a floor. Further, relative to a vertical more than 50% of the radiant flux of the system may propagate in a direction away from the floor. In specific embodiments, relative to a vertical the percentage of the radiant flux of the system which may propagate in a direction away from the floor may be at least 60%, such as at least about 80%, like even more especially at least about 90% of the radiant flux of the system (radiation).

Hence, the method may comprise configuring the system such that the radiant flux of the radiation generating system particularly irradiates the upper part of a space. This may facilitate improved disinfection in those areas with no human presence, while remaining safe for human presence in the lower part of spaces.

In embodiments, the space in which the radiation generating system is configured may comprise a ceiling. In such embodiments, the radiation generating system may be configured to irradiate with the system radiation at least part of the ceiling.

Hence, the method may comprise a configuration in which the upper part of rooms is irradiated more than the lower part of rooms. This may facilitate improved disinfection in those areas with no human presence, while remaining safe for human presence in the lower part of spaces.

In embodiments, the radiation generating system may comprise additional reflectors of lamellae, which will further reflect and redirect generated system radiation. Hence, such embodiments may facilitate the accommodation of stringent safety requirements.

### BRIEF DESCRIPTION OF THE DRAWINGS

Embodiments of the invention will now be described, by way of example only, with reference to the accompanying schematic drawings in which corresponding reference symbols indicate corresponding parts, and in which:
Fig. 1A-C schematically depict embodiments of the system.
Fig 2A-B schematically depict further embodiments of the system.
Fig 3A-B schematically depict embodiments of the method.

The schematic drawings are not necessarily to scale.

### DETAILED DESCRIPTION OF THE EMBODIMENTS

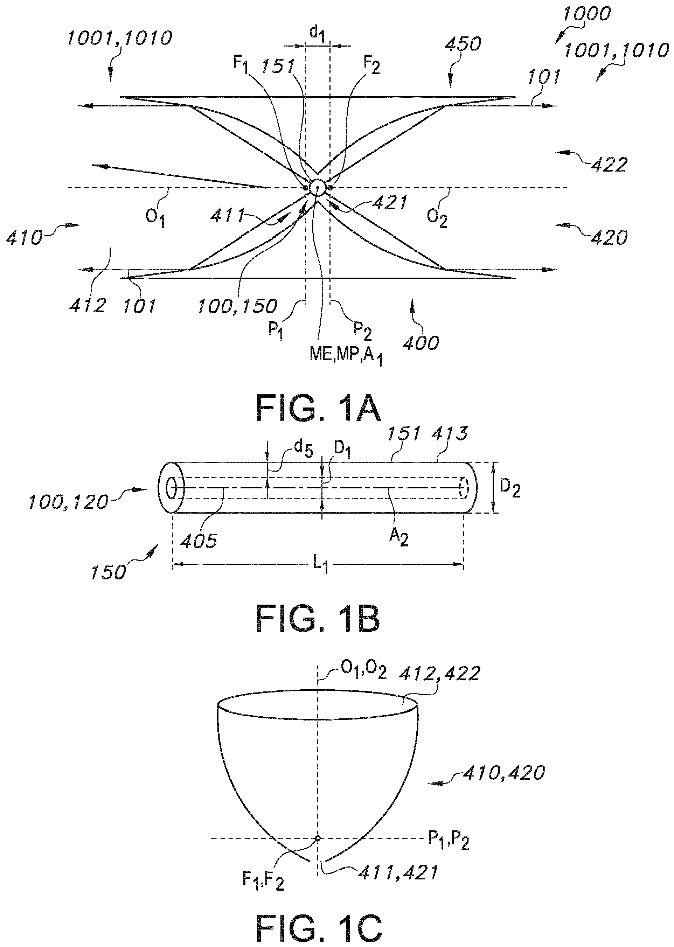

Fig. 1A-C schematically depict a radiation generating system 1000 comprising (i) an optical element assembly 400, and (ii) a light generating device 100. The light generating device 100 is configured to generate device radiation 101 having one or more wavelengths selected from the wavelength range of 100-380 nm. The light generating device 100 comprises an elongated element 150 having an element length L1. The elongated element 150 comprises one or more radiation emitting faces 151, over at least part of the element length L1. During operation of the light generating device 100 the device radiation 101 emanates from the radiation emitting faces 151. The optical element assembly 400 comprises a first hollow reflective element 410 and a second hollow reflective element 420. The hollow reflective elements 410,420 each have a first end 411,421 and a second end 412,422. The hollow reflective elements 410,420 each taper from the second end 412,422 to the first end 411,421. The hollow reflective elements 410,420 each have an optical axis O₁,O₂. The hollow reflective elements 410,420 each comprise one or more focal points F₁,F₂. The focal points F₁,F₂ are each configured in a plane P₁,P₂ perpendicular to the optical axis O₁,O₂. The first ends 411,421 of the hollow reflective elements 410,420 are directed to each other or coincide. The planes P₁,P₂ do not coincide. The elongated element 150 is (i) configured at least partly coinciding with the optical axes O₁,O₂ or (ii) configured between the first ends 411,421 and perpendicular to the optical axes O₁,O₂. Especially, an axis of elongation A₂ of the elongated element 150 may essentially coincide with the optical axes O₁,O₂. The radiation generating system 1000 is configured to generate system radiation 1001 comprising at least part of the device light 101 escaping from at least one of the hollow reflective elements 410,420.

Further, Fig. 1A-C schematically depict embodiments wherein the light generating device 100 comprises a discharge source 120 based on one or more of zinc, mercury, cadmium, xenon, and krypton. The discharge source 120 further comprises a gas composition 405. Especially, in embodiments, the discharge source 120 is based on zinc. At least 30% of the spectral power in the wavelength range of 200-500 nm is provided by spectral power in the wavelength range of 200-230 nm.

In further such embodiments, the elongated element 150 comprises a tubular element comprising an external cylindrical wall 413 having an internal diameter D₁ and an external diameter D₂. One or more radiation emitting faces 151 are comprised by the cylindrical wall 413.

Yet further, Fig. 1A-C schematically depict embodiments wherein each of the hollow reflective elements 410,420 have one or more parabola-like cross-sectional shapes. The planes P₁,P₂ have a mutual distance d₁.

In further such embodiments, at least one of the mentioned parabola-like cross-sectional shapes have the shape of a cross-section of a compound parabolic reflector.

In yet further such embodiments, the hollow reflective elements 410,420 are comprised by one or more thermally conductive bodies 450. Especially, in embodiments, the hollow reflective elements 410,420 are comprised by one or more aluminum bodies 450.

In embodiments, the light generating device 100 and at least one of the hollow reflective elements 410,420 are configured to generate a beam 1010 of system radiation 1001 having in at least one direction a full width half maximum of at maximum 35°.

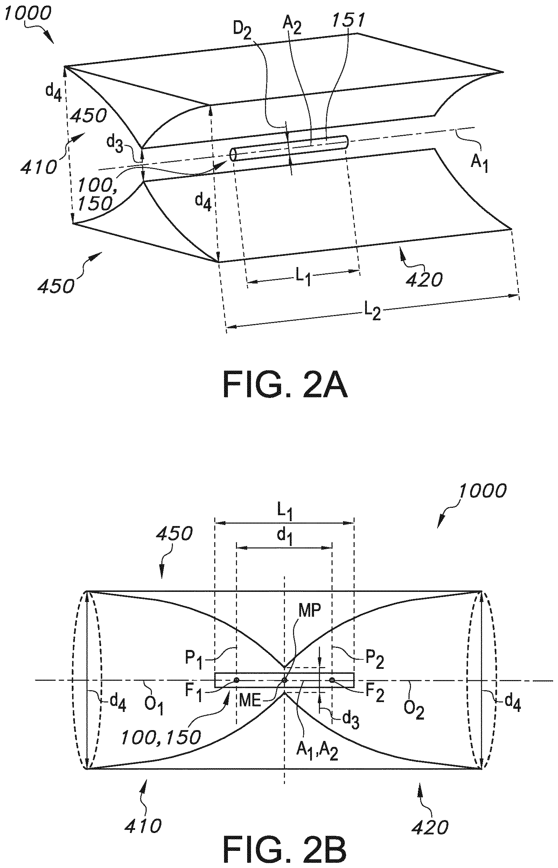

Fig. 1A may e.g. be a cross-sectional shape of the embodiments schematically depicted in Figs. 2A and 2B.

Fig. 1A also depicts a middle MP between the planes P₁,P₂ and a middle ME of the elongated element 150. Reference A₂ indicated an axis of elongation of the elongated element 150. Reference A₁ may refer to an axis of elongation of the optical element assembly 400.

Fig. 1B schematically depicts a perspective view of an embodiment of the elongated element 150, such as a discharge vessel.

Fig. 1C schematically depicts one of the hollow reflective elements 410,420. Hence, all references are double as essentially all features may apply to both hollow reflective elements 410,420. Here, by way of example a hollow reflective element is depicted which has a substantially circular cross-section, see e.g. also Fig. 2B. However, as shown in Fig. 2, along one direction, the hollow reflective element may be elongated. In such embodiments, a cross-section (parallel to the optical axis) may essentially be rectangular.

Fig. 2A schematically depicts specific embodiments wherein the elongated element is configured between the first ends 411,421 and perpendicular to the optical axes O₁,O₂. Further, the optical element assembly is elongated having an axis of elongation A₁ and a second length L2. The elongated element 150 and the axis of elongation A₁ are configured parallel, wherein 0.9≤L2/L1≤5.

In further such embodiments, each of the hollow reflective elements 410,420 may have a parabola-like cross-sectional shape. The parabola-like cross-sectional shapes have a mutual distance, which tapers from the shortest distance d₃ to the longest distance d₄ between the parabola-like cross-sectional shapes. The planes P₁,P₂ have a mutual distance d₁, wherein 0.8≤d₁/D₂≤1.25. In embodiments, the shortest distance may be shortest distance d₃ between opposite sides of the hollow reflective element at the first end 411 or 421. This may in embodiments be a diameter, such as in Fig. 2B, but it may also be a distance like in Fig. 2A. In embodiments, the longest distance d₄ may be shortest distance between opposite sides of the hollow reflective element at the second end 412 or 422. This may in embodiments be a diameter, such as in Fig. 2B, but i may also be a distance like in Fig. 2A.

Fig. 2B schematically depicts specific embodiments wherein the elongated element 150 is configured at least partly coinciding with the optical axes O₁,O₂.

In further such embodiments, each of the hollow reflective elements 410,420 have two orthogonal parabola-like cross-sectional shapes. The parabola-like cross-sectional shapes have a mutual distance, which tapers from the shortest distance d₃ to the longest distance d₄ between the parabola-like cross-sectional shapes. The planes P₁,P₂ have a mutual distance d₁. A middle MP between the planes P₁,P₂ and a middle ME of the elongated element 150 are configured within distance of each other at maximum 0.1^{∗}d₁, wherein 1.5≤L1/d₁≤2.5.

Referring to Figs 2A-2B, for a single hollow reflective element, there may be a single focal point, see Fig. 2B, wherein each hollow reflective element may comprise a single focal point. However, there may also be more than one focal points, like a line (comprising two or more focal points), such as may be in the case of an elongated hollow reflective element, like schematically depicted in Fig. 2A.

Reference A₁ may refer to an axis of elongation of the optical element assembly 400. Such axis A₁ may be available in the embodiment schematically depicted in Fig. 2a, but not in the embodiment schematically depicted in Fig. 2B.

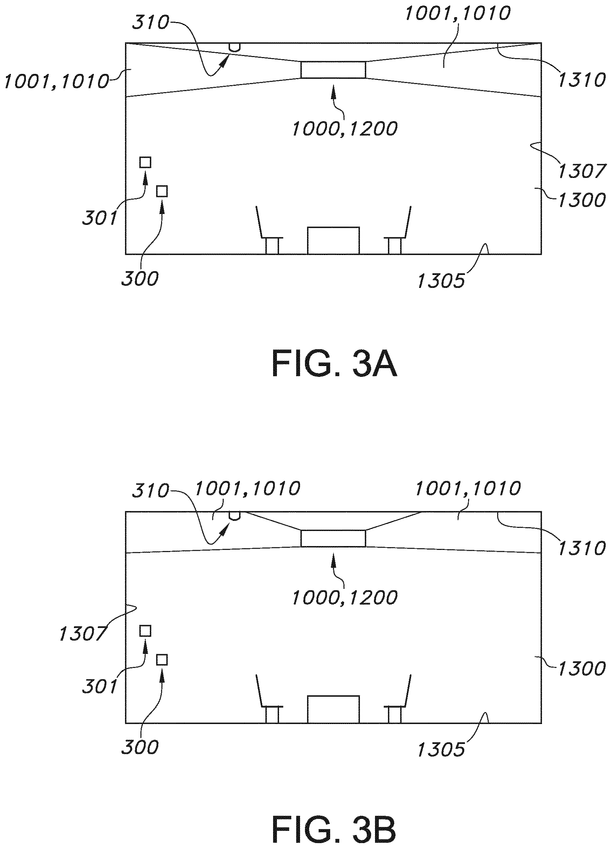

Fig. 3A-B schematically depicts an application of a method for treating a gas or a surface (external of the radiation generating system 1000). The method comprises providing the system radiation 1001 to the gas or the surface with the radiation generating system 1000. Hence, Fig. 3A-B schematically depict embodiments of a disinfection device 1200 comprising the radiation generating system 1000.

In such embodiments, the system 1000 is configured in a space 1300 comprising a floor 1305. Relative to a vertical more than 50% of a radiant flux of the system radiation 1001 propagates in a direction away from the floor 1305. The radiation generating system 1000 is configured to generate a beam 1010 of system radiation 1001. The beam 1010 of system radiation 1001 has in a vertical direction a full width half maximum of at maximum 35°.

In further such embodiments, the space 1300 comprises a ceiling 1310 and at least one wall 1307. The radiation generating system 1000 is configured to irradiate with the system radiation 1001 at least part of the ceiling 1310.

In yet further such embodiments, reference 301 indicates a user interface which may be functionally coupled with the control system 300 comprised by or functionally coupled to the radiation generating system 1000.

Fig. 3B schematically depicts a specific embodiment wherein the light generating device 100 and at least one of the hollow reflective elements 410,420 are configured to generate a beam of system radiation 1001 having in at least one direction a full width half maximum of at maximum 35°.

The term "plurality" refers to two or more.

The terms "substantially" or "essentially" herein, and similar terms, will be understood by the person skilled in the art. The terms "substantially" or "essentially" may also include embodiments with "entirely", "completely", "all", etc. Hence, in embodiments the adjective substantially or essentially may also be removed. Where applicable, the term "substantially" or the term "essentially" may also relate to 90% or higher, such as 95% or higher, especially 99% or higher, even more especially 99.5% or higher, including 100%.

The term "comprise" also includes embodiments wherein the term "comprises" means "consists of'.

The term "and/or" especially relates to one or more of the items mentioned before and after "and/or". For instance, a phrase "item 1 and/or item 2" and similar phrases may relate to one or more of item 1 and item 2. The term "comprising" may in an embodiment refer to "consisting of" but may in another embodiment also refer to "containing at least the defined species and optionally one or more other species".

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

The devices, apparatus, or systems may herein amongst others be described during operation. As will be clear to the person skilled in the art, the invention is not limited to methods of operation, or devices, apparatus, or systems in operation.

It should be noted that the above-mentioned embodiments illustrate rather than limit the invention, and that those skilled in the art will be able to design many alternative embodiments without departing from the scope of the appended claims.

In the claims, any reference signs placed between parentheses shall not be construed as limiting the claim.

Use of the verb "to comprise" and its conjugations does not exclude the presence of elements or steps other than those stated in a claim. Unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

The article "a" or "an" preceding an element does not exclude the presence of a plurality of such elements.

The invention may be implemented by means of hardware comprising several distinct elements, and by means of a suitably programmed computer. In a device claim, or an apparatus claim, or a system claim, enumerating several means, several of these means may be embodied by one and the same item of hardware. The mere fact that certain measures are recited in mutually different dependent claims does not indicate that a combination of these measures cannot be used to advantage. In yet a further aspect, the invention (thus) provides a software product, which, when running on a computer is capable of bringing about (one or more embodiments of) the method as described herein.

The invention also provides a control system that may control the device, apparatus, or system, or that may execute the herein described method or process. Yet further, the invention also provides a computer program product, when running on a computer which is functionally coupled to or comprised by the device, apparatus, or system, controls one or more controllable elements of such device, apparatus, or system.

The invention further applies to a device, apparatus, or system comprising one or more of the characterizing features described in the description and/or shown in the attached drawings. The invention further pertains to a method or process comprising one or more of the characterizing features described in the description and/or shown in the attached drawings.

The various aspects discussed in this patent can be combined in order to provide additional advantages. Further, the person skilled in the art will understand that embodiments can be combined, and that also more than two embodiments can be combined. Furthermore, some of the features can form the basis for one or more divisional applications.

## Claims

1. A radiation generating system (1000) comprising (i) an optical element assembly (400), and (ii) a light generating device (100), wherein:
- the light generating device (100) is configured to generate device radiation (101) having one or more wavelengths selected from the wavelength range of 100-380 nm; the light generating device (100) comprises an elongated element (150) having an element length (L1), wherein the elongated element (150) comprises one or more radiation emitting faces (151), over at least part of the element length (LI), from which during operation of the light generating device (100) the device radiation (101) emanates;
- the optical element assembly (400) comprises a first hollow reflective element (410) and a second hollow reflective element (420), wherein for the hollow reflective elements (410,420) applies: (a) the hollow reflective element (410,420) has a first end (411,421) and a second end (412,422); (b) the hollow reflective element (410,420) tapers from the second end (412,422) to the first end (411,421); (c) the hollow reflective element (410,420) has an optical axis (O₁,O₂); and (d) the hollow reflective element (410,420) comprises one or more focal points (F₁,F₂), configured in a plane (P₁,P₂) perpendicular to the optical axis (O₁,O₂);
- the first ends (411,421) of the hollow reflective elements (410,420) are directed to each other or coincide, and the planes (P₁,P₂) do not coincide;
- the elongated element (150) is (i) configured at least partly coinciding with the optical axes (O₁,O₂) or (ii) configured between the first ends (411,421) and perpendicular to the optical axes (O₁,O₂);
- the radiation generating system (1000) is configured to generate system radiation (1001) comprising at least part of the device light (101).

2. The radiation generating system (1000) according to claim 1, wherein the light generating device (100) comprises a discharge source (120) based on one or more of zinc, mercury, cadmium, xenon, and krypton; and wherein the elongated element (150) comprises a tubular element comprising an external cylindrical wall (413) having an external diameter (D₂), wherein the one or more radiation emitting faces (151) are comprise by the cylindrical wall (413).

3. The radiation generating system (1000) according to claim 2, wherein the discharge source (120) is based on zinc; and wherein at least 30% of the spectral power in the wavelength range of 200-500 nm is provided by spectral power in the wavelength range of 200-230 nm.

4. The radiation generating system (1000) according to any one of the preceding claims 1-3, wherein the optical element assembly (400) is elongated having an axis of elongation (A₁) and having a second length (L2), wherein the elongated element (150) and the axis of elongation (A₁) are configured parallel, and wherein 0.9≤L2/L1≤5.

5. The radiation generating system (1000) according to claim 4, wherein each of the hollow reflective elements (410,420) have a parabola-like cross-sectional shape.

6. The radiation generating system (1000) according to any one of the preceding claim 4-5 and according to claim 2, wherein the planes (P₁,P₂) have a mutual distance (di), wherein 0.8≤d₁/D₂≤1.25.

7. The radiation generating system (1000) according to any one of the preceding claims 1-3, wherein each of the hollow reflective elements (410,420) have two orthogonal parabola-like cross-sectional shapes.

8. The radiation generating system (1000) according to claim 7, wherein the planes (P₁,P₂) have a mutual distance (di), wherein a middle (MP) between the planes (P₁,P₂) and a middle (ME) of the elongated element (150) are configured within distance of each other of at maximum 0.1^{∗}d₁, and wherein 1.5≤L1/d₁≤2.5.

9. The radiation generating system (1000) according to any one of claims 5 and 7, wherein at least one of the mentioned parabola-like cross-sectional shapes have the shape of a cross-section of a compound parabolic reflector.

10. The radiation generating system (1000) according to any one of the preceding claims, wherein the hollow reflective elements (410,420) are comprised by one or more thermally conductive bodies (450).

11. The radiation generating system (1000) according to any one of the preceding claims, wherein the hollow reflective elements (410,420) are comprised by one or more aluminum bodies (450).

12. The radiation generating system (1000) according to any one of the preceding claims, wherein the light generating device (100) and at least one of the hollow reflective elements (410,420) are configured to generate a beam (1010) of system radiation (1001) having in at least one direction a full width half maximum of at maximum 35°.

13. A method for treating a gas or a surface with the radiation generating system (1000) according to any one of the preceding claims 1-12, the method comprising providing the system radiation (1001) as defined in any one of the preceding claims 1-12 to the gas or the surface with the radiation generating system (1000).

14. The method according to claim 13, wherein the system (1000) is configured in a space (1300) comprising a floor (1305) wherein relative to a vertical more than 50% of a radiant flux of the system light (1001) propagates in a direction away from the floor (1305); wherein the radiation generating system (1000) is configured to generate a beam (1010) of system radiation (1001) having in a vertical direction a full width half maximum of at maximum 35°.

15. The method according to claim 14, wherein the space comprises a ceiling (1310), wherein the system (1000) is configured to irradiate with the system radiation (1001) at least part of the ceiling (1310).
